# EUROPEAN PATENT SPECIFICATION

(11) **EP 1 196 120 B1**
(45) Date of publication and mention of the grant of the patent: **03.11.2004**
(21) Application number: 00942579.4
(22) Date of filing: 21.06.2000
(51) Int. Cl.: A61F 5/00, A63B 29/00

(54) **HEAD SUPPORT**
KOPFSTÜTZE
TETIERE

(30) Priority: 21.06.1999 NZ 33635899
(43) Date of publication of application: 17.04.2002
(73) Proprietor: Poole, Darrell Maitland, RD 6 Tauranga (NZ)
(72) Inventor: Poole, Darrell Maitland, RD 6 Tauranga (NZ)
(74) Representative: Alexander, Thomas Bruce
(86) International application number: PCT/NZ2000/000106
(87) International publication number: WO 2000/078254

(56) References cited:
- DE-A1- 2 753 116
- NL-C- 122 871
- US-A- 4 280 490
- US-A- 4 401 111
- US-A- 4 527 833
- US-A- 4 643 174
- US-A- 5 248 293
- US-A- 5 292 043
- US-A- 5 314 404
- US-A- 5 891 068

## Description

### FIELD OF INVENTION

The present invention relates to improvements in or relating to head supports. More particularly but not exclusively it relates to head or neck supports which support the head or neck in a tilted back position, and/or to provide support for viewing objects above the horizontal.

### BACKGROUND TO THE INVENTION

In activities such as climbing, fruit picking, plastering, painting or attending air shows or the like where a person is, for long durations, required or desired to have his/her head tilted back so as to allow them to look upwardly or at least above the horizontal, such tilting of the head can only be sustained for a limited period of time. Although muscle strength to support a head in a tilted position does vary from person to person, after a while anyone will start to feel the strain in having to maintain the head in such a condition. In activities such as climbing where a belayer is positioned below the climber and whose function is to maintain the tension in the rope which is attached to the climber, it is essential that this person keeps a close eye on the progress of the climber as he/she ascends or descends. Failure to maintain a substantially constant eye on the progress of the climber could result in a sufficient amount of slack in the rope which if the climber falls, could result in a fall, which may cause injury or death.

It is an object of the present invention to provide a head support which will at least go some way towards providing a means of supporting the head or neck of a person in a tilt back condition or which will at least provide the public with a useful choice.

As used herein, "the horizontal" describes that field of view of the eyes in the plane substantially perpendicular to the body of the user, which is considered as being in the "upright" position. The "upright" position may include a position which is not strictly vertical or upright as in conventionally understood. For example, when in a standing, leaning back, or sitting position, the body is defined as being in the ''upright'' position. The "horizontal" view is that with the eyes facing substantially straight ahead. In viewing an object "above the horizontal" the head and/or neck will require tilting backwards somewhat from this position.

"Harness" as used herein may also include a backpack or other system capable of being worn by or attached to a user, and supporting or appending to or functioning as a head or neck support as described.

U.S. 5,891,068 discloses a head hyperextension-type orthotic device for treating a contracture of neck muscular fibres. The orthotic device comprises a curved stiff bar and a series of elastic cylinders or neck bolsters which are sequentially installed between the bar and a back region of a user's head to move the head forwardly to counteract the hyperextension.

### BRIEF DESCRIPTION OF THE INVENTION

Accordingly, the first aspect of the present invention consists in a head support (1) comprising:
a) a harness locatable to the upper body portions of a person,
b) a rigid member extending from a region of said harness stabilised in respect of and by the use of the harness relative to the body wherein, when in use, said harness is attached to the body of a person, said rigid member extending therefrom to provide a support region thereof wherein when said person is in an upright position, and the head is tilted backwards to allow viewing of an object above the horizontal, said support region comes in contact with at least part of the back of the head and provides at least vertical support to the head,
said rigid member having a vertically extending portion which, in use extends substantially vertically and parallel with said upright position of said user, and an outwardly curved extending portion which extends, in use away from said vertically extending portion, outwardly from the body of a user wherein at or near a distal end of said outwardly curved extending portion said head support region is provided, said rigid member being an elongate member having a first distal end defining a lower end of said vertically extending portion and a second distal end defining an outer end of said outwardly curved extending portion.

Preferably, said rigid member is secured to said harness at said vertically extending portion thereof.

Preferably, said rigid member is pressed against is the upper spine region of the back of the user.

Preferably, said rigid member is an extrusion, or is of a single elongate sheet material shaped, moulded or fabricated to provide said vertically extending region and said outwardly curved extending region thereof.

Preferably, said head supporting region projects laterally from said outwardly curved extending portion to additionally provide support to the sides of the head.

In a further aspect, the harness is a back pack wherein said vertically extending region has a region engaging with a head support receiving device located at least adjacent to a top of the back pack.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples.

### DESCRIPTION OF PREFERRED EMBODIMENT

One preferred form of the present invention will now be described with reference to the accompanying drawings in which:
Figure 1 is a perspective view of the back of a user of the present invention illustrating the head support located with the user in the preferred condition,
Figure 2 is a front view in direction AA of figure 3 of the head support of the present invention,
Figure 3 is a perspective view of the head support as shown in figure 2,
Figure 4 is a view from the back of an alternative and preferred form of the head support of the present invention
Figure 5 illustrates the rigid member of the head support in association with a lateral stabilising means with which the straps of the harness can locate.
Figure 6 illustrates a side perspective view of a preferred form of the rigid member of the head support.
Figure 7 illustrates a front view in the direction AA of Figure 3 of the preferred form of Figure 6.
Figure 8 illustrates a front view in the direction AA of Figure 3 of an alternative form of the invention, being a back pack.
Figure 9 illustrates the invention in use by, for example, a belayer
Figure 10 illustrates the invention in use by, for example, a climber.
Figure 11 illustrates the invention in use by, for example, a decorator or painter.

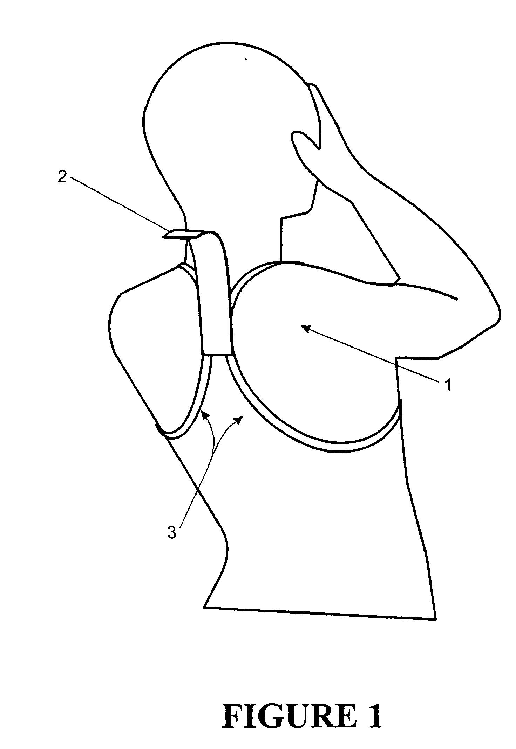

With reference to figure 1 there is shown a head support 1 which consists of a rigid member 2 and a harness 3 located or secured with said rigid member.

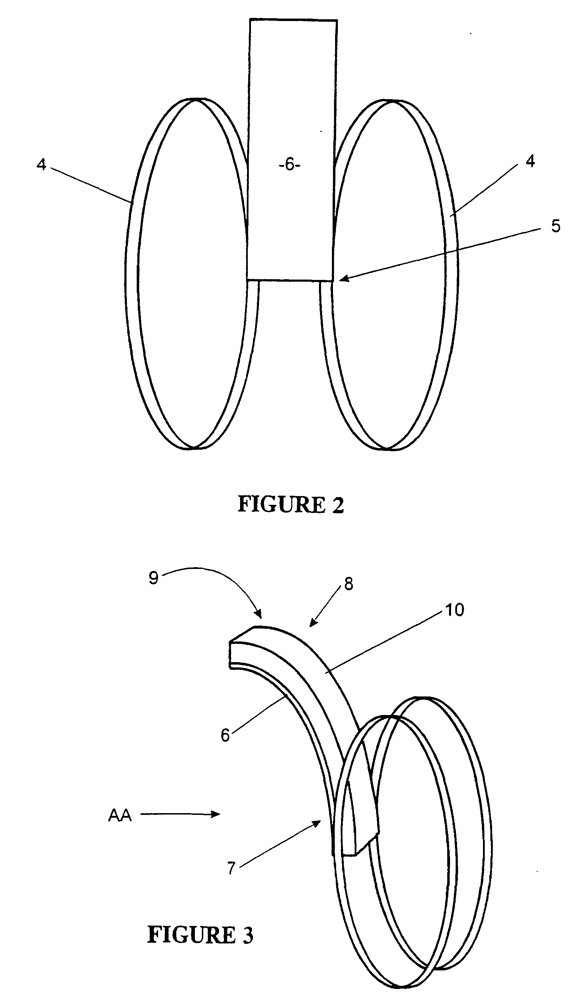

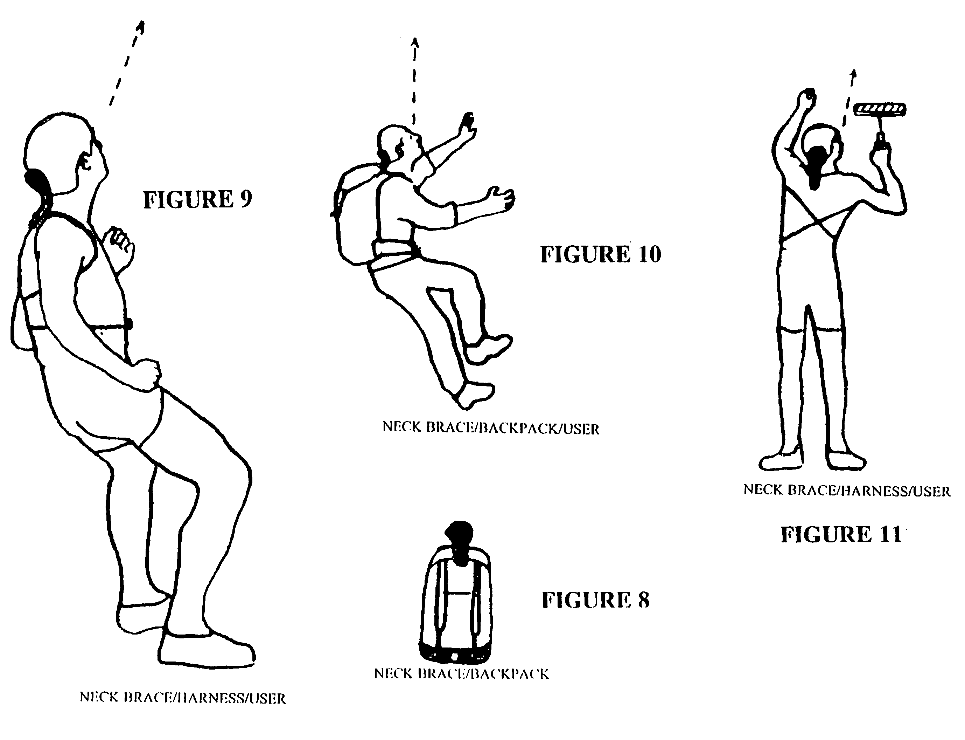

The rigid member and harness are adapted to be located to the user of the head support of the present invention such that when the user is standing upright and tilts his/her head backwards, there is a region of said rigid member 2 with which the tilted back head can contact and be provided with a vertical support thereby. It is to be noted that the support of the present invention may simply provide neck support for viewing above the horizontal rather than head support, without departing from the scope of the present invention. With reference to figures 2, 9, 10 and 11 there is shown a user of the head support with his head tilted backwards and a part of the head locating against a portion of the rigid member to provide the user with a comfortable and vertical support for the head or neck.

In reference to figures 2 and 3 in the preferred form of the present invention the head support consists of harness which is locatable about the upper body regions of a user of the present invention. The harness is of a configuration which can provide at least vertical stability to the rigid member. It is to be noted that the "harness" can also include a backpack without departing from the scope of the invention. In figure 2 in the preferred form there is shown a harness which consists of two shoulder straps 4 which are locatable over the shoulder and underneath the armpits to return back to a region of attachment 5 between the rigid member 6 and the harness 3. In the preferred form the region of attachment is at or towards a lower distal end of the rigid member 6 of the present invention however it is envisaged by the inventor that such location points can vary. With reference to figure 1 there is illustrated the shoulder straps 4 running from the region of attachment to the rigid member, over the shoulders under the arms and back towards the region of securement. It will be appreciated by a person skilled in the art that such shoulder straps may be provided with buckles and/or adjustment means to allow for the head support of the present invention to be reconfigured to allow users of different body shapes and sizes to use the particular head support. Furthermore, as discussed the shoulder straps may be those of a backpack configuration.

As indicated in Figures 2, 3, 6 and 7 in the preferred form the rigid member 6 is preferably made up from a sheet material such as a moulded plastic, composite or metallic or non-metallic material. The rigid member 6 preferably consists of a vertically extending region 7 and an outwardly extending region 8. The vertically extending region 7 is provided and adapted in association with the harness to lie parallel to and in engagement with the back of a user of the head support of the present invention. Preferably said vertically extending region 7 is of sufficient length to be pressed when said harness is engaged to the user, against the back of the user to provide some stable support in respect of the user of the present invention.

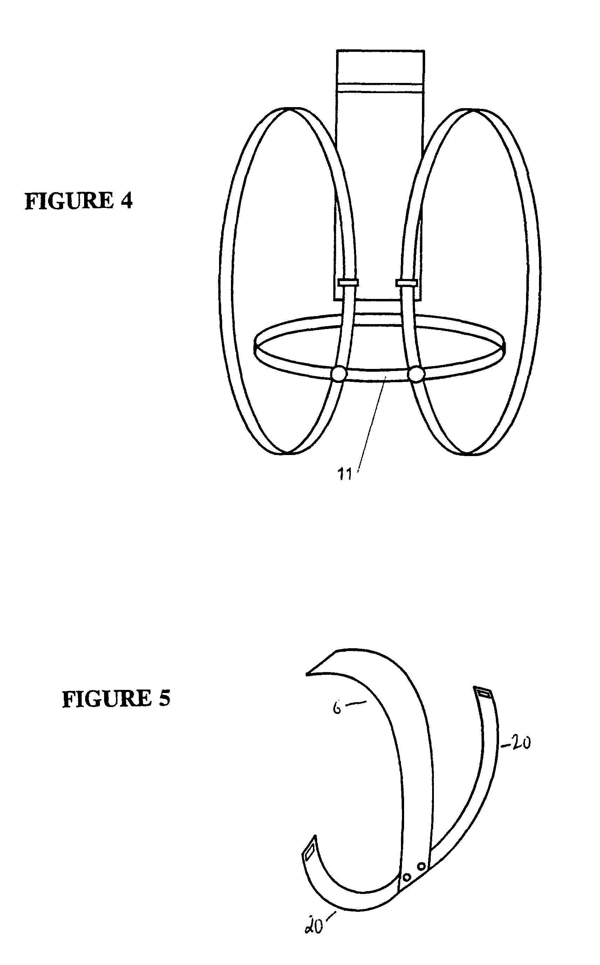

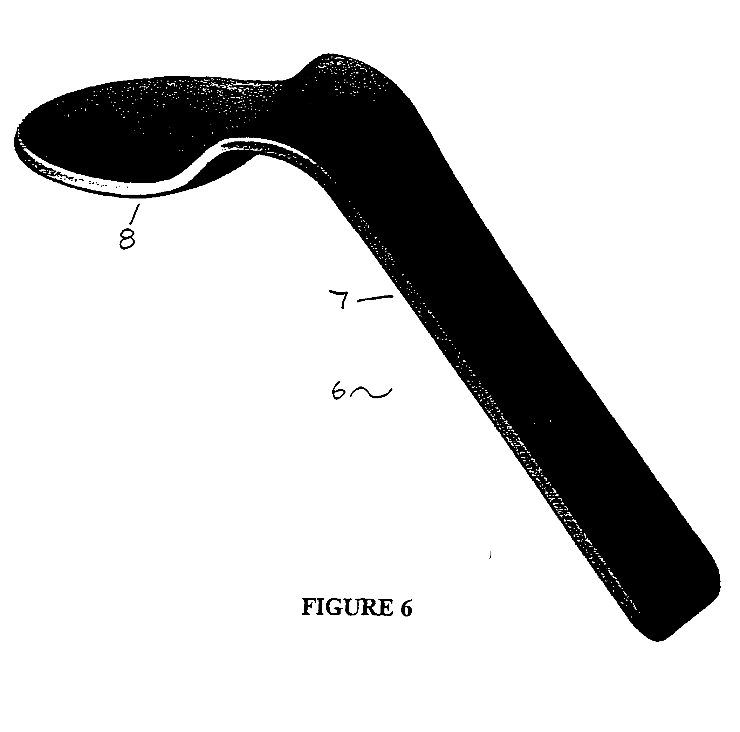

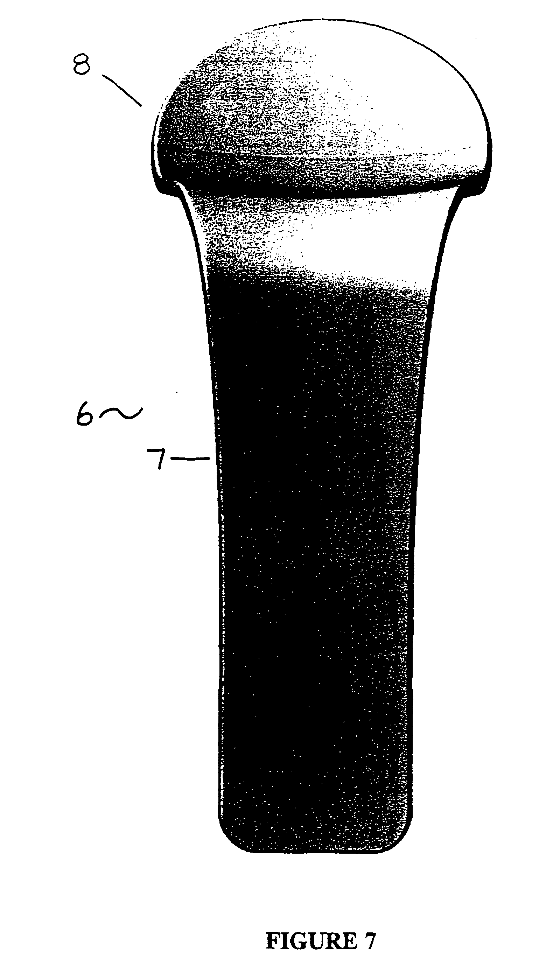

The outwardly extending portion 8 of the rigid member has a head contacting region 9 with which, when said head support is in use, the tilted back head of a user can comfortably located. As indicated in Figures 6 and 7, the outwardly extending portion may be concave to allow engagement of the head as shown in Figures 3 and 4, for comfort in the wearing of the head support of the present invention there is preferably provided a padded surface 10 on that side of the rigid member which is to be in contact with the body of the user.

Figure 4 shows and alternative configuration or an additional configuration to the harness of the head support of the present invention. To encourage the rigid means to remain in stable and close contact with the back of a user a further portion of the harness is provided to encompass the chest or waist of the user of the device. This portion 11 may be attached to the shoulder straps 4 and/or be attached to the rigid member of the present invention. Again a person skilled in the art will appreciate that this strap 11 may be provided with a buckle and/or strap length adjusting means to allow a particular head support of the present invention to be adapted for use by different sized and shaped people.

For further support to the rigid means of the present invention a lateral stabiliser may be rigidly attached or form part of the rigid member 6. With reference to figure 5 such a lateral stabiliser may for example be arms 20 which extend outwardly (and preferably upwardly) from a region of the rigid member 6. Preferably the region from which the lateral stabilisers extend is a lower or lower most region of the rigid member. The lateral stabilisers are preferably made of a material similar to that of the rigid member or may alternatively be made from any other material and which although preferably deformable, does have its own inherent rigidity. In the preferred form the lateral stabilisers locate at the distal ends with the shoulder straps 4. The shoulder straps will tend to draw the lateral stabiliser to conform with the upper back and shoulder shape of the user of the present invention and hence in the preferred form such lateral stabilisers are of a sheet material or strip wherein the plane of the sheet or strip is in a general parallel direction with the back of the user. Such an orientation of the plane of the lateral stabilisers will allow for it to be conformed or at least in part contoured with the back of the user.

Preferably at the distal ends of the lateral stabiliser there is the provision of the slot through which the shoulder straps 4 can extend while still allowing the distal ends of the lateral stabilisers to have some association with said shoulder straps.

## Claims

1. A head support (1) comprising:
a) a harness (3) locatable to the upper body portions of a person,
b) a rigid member (2)
extending from a region of said harness (3) stabilised in respect of and by
the use of the harness relative to the body
wherein, when in use, said harness (3) is attached to the body of a person, said rigid member (2) extending therefrom to provide
a support region (9) thereof
which when said person is in an upright position, and the head is tilted backwards to allow viewing of an object above the horizontal, said support region (9) comes in contact with at least part of the back of the head and provides at least vertical support to the head, said rigid member (2) having
a vertically extending portion (7)
which, in use extends substantially vertically and parallel with said upright position of said user, said rigid member (2) being
an elongate member having
a first distal end defining a lower end of said vertically extending portion (7) **characterized in that** said rigid member (2) comprises
an outwardly curved extending portion (8)
which extends, in use away from said vertically extending portion (7), outwardly from the body of a user wherein at or near a distal end of said outwardly curved extending portion (8) said head support region (9) is provided,
said elongate member having a second distal end defining an outer end of said outwardly curved extending portion (8).

2. A head support (1) as claimed in claim 1 wherein said rigid member (2) secured to said harness (3) at said vertically extending portion (7) thereof.

3. A head support (1) as claimed in claim 1 wherein said rigid member (2) pressed against the upper spine region of the back of the user.

4. A head support (1) as claimed in claim 1 wherein said rigid member (2) is extrusion or is of a single elongate sheet material shaped, moulded or fabricated provide said vertically extending region (7) and said outwardly curved extending portion (8) thereof.

5. A head support (1) as claimed in claim 4 wherein said head supporting region (9) projects laterally from said outwardly curved extending portion (8) to additional provide support to the sides of the head.

6. A head support (1) as claimed in claim 1 wherein said harness (3) is a back pack and said vertically extending region (7) has a region engaging with a head support receiving device located at least adjacent to a top of the back pack.

## Patentansprüche

1. Kopfstütze (1), umfassend:
a) ein Geschirr (3), welches an den oberen Körperteilen einer Person angeordnet werden kann,
b) ein steifes Element (2),
welches sich von einem Bereich des Geschirres (3) aus erstreckt, gegenüber dem Geschirr und durch dessen
Verwendung gegenüber dem Körper stabilisiert ist,
wobei das Geschirr (3) beim Verwenden am Körper einer Person angebracht ist, von welchem aus sich das steife Element (2) erstreckt um
einen Stütz-Bereich (9) davon vorzusehen,
so dass, wenn die Person sich in einer aufrechten Position befindet, und der Kopf nach hinten geneigt ist, um das Betrachten eines Objektes oberhalb der Horizontalen zu ermöglichen, dieser Stütz-Bereich (9) mit wenigstens einem Teil des Hinterteils des Kopfes in Kontakt kommt, und dem Kopf wenigstens vertikale Abstützung gibt, wobei das steife Element (2)
einen sich vertikal erstreckenden Abschnitt (7) aufweist, welcher sich im Gebrauch im Wesentlichen vertikal und parallel zu der aufrechten Position des Anwenders erstreckt, und wobei das steife Element (2)
ein langgestrecktes Element ist, welches
ein erstes distales Ende aufweist, welches ein unteres Ende des sich vertikal erstreckenden Abschnittes (7) definiert, **dadurch gekennzeichnet, dass** das steife Element (2)
einen auswärts gekrümmten abstehenden Abschnitt (8) umfasst,
welcher im Gebrauch von dem sich vertikal erstreckenden Abschnitt (7) weg, auswärts von dem Körper eines Anwenders absteht, wobei der Kopf-Stütz-Bereich (9) an oder nahe bei einem distalen Ende des auswärts gekrümmten abstehenden Abschnittes (8) vorgesehen ist,
wobei das langgestreckte Element ein zweites distales Ende aufweist, welches ein äußeres Ende des sich auswärts erstreckenden gekrümmten Abschnittes (8) definiert.

2. Kopfstütze (1) nach Anspruch 1, wobei das steife Element (2) an dem Geschirr (3) an dessen sich vertikal erstreckenden Abschnitt (7) befestigt ist.

3. Kopfstütze (1) nach Anspruch 1, wobei das steife Element (2) gegen den oberen Wirbelsäulen-Bereich des Rückens des Anwenders gepresst wird.

4. Kopfstütze (1) nach Anspruch 1, wobei das steife Element (2) ein Extrusionsteil ist, oder aus einem einzigen länglichen Tafel-Material besteht, welches gestaltet, geformt oder hergestellt ist, um dessen sich vertikal erstreckenden Bereich (7) und den auswärts gekrümmten Bereich (8) vorzusehen.

5. Kopfstütze (1) nach Anspruch 4, wobei der Kopf-stützende Bereich (9) seitlich von dem auswärts gekrümmten abstehenden Abschnitt (8) vorsteht, um zusätzlich den Seiten des Kopfes Abstützung zu geben.

6. Kopfstütze (1) nach Anspruch 1, wobei das Geschirr (3) ein Rucksack ist, und der sich vertikal erstreckenden Bereich (7) einen Bereich aufweist, welcher in eine wenigstens benachbart zu einem Oberteil des Rucksackes angeordnete Kopfstütze-Aufnahme-Vorrichtung eingreift.

## Revendications

1. Support de tête (1) comprenant :
a) un harnais (3) pouvant se situer sur les parties supérieures du corps d'une personne,
b) un élément rigide (2)
s'étendant à partir d'une région dudit harnais (3) stabilisée par rapport à et grâce à l'utilisation du harnais par rapport au corps,
dans lequel, lors de l'utilisation, ledit harnais (3) est fixé au corps d'une personne, ledit élément rigide (2) s'étendant à partir de celui-ci afin de fournir
une région de support (9) de celui-ci
qui, lorsque ladite personne est dans une position verticale, et que la tête est inclinée vers l'arrière pour permettre l'observation d'un objet situé au dessus de l'horizontale, ladite région de support (9) vient en contact avec au moins une partie de l'arrière de la tête et fournit au moins un support vertical à la tête, ledit élément rigide (2) ayant
une partie s'étendant verticalement (7)
qui, lors de l'utilisation s'étend sensiblement verticalement et parallèlement à ladite position verticale dudit utilisateur, ledit élément rigide (2) étant
un élément allongé comprenant
une première extrémité distale définissant une extrémité inférieure de ladite partie s'étendant verticalement (7), **caractérisé en ce que** ledit élément rigide (2) comprend
une partie incurvée s'étendant vers l'extérieur (8)
qui s'étend, lors de l'utilisation, à distance de ladite partie s'étendant verticalement (7), vers l'extérieur du corps d'un utilisateur, dans lequel au niveau de ou à proximité d'une extrémité distale de ladite partie incurvée s'étendant vers l'extérieur (8), ladite région de support de tête (9) est prévue, une seconde extrémité distale définissant une extrémité externe de ladite partie incurvée s'étendant vers l'extérieur (8).

2. Support de tête (1) selon la revendication 1, dans lequel ledit élément rigide (2) est fixé audit harnais (3) au niveau de ladite partie s'étendant verticalement (7) de celui-ci.

3. Support de tête (1) selon la revendication 1, dans lequel ledit élément rigide (2) est comprimé contre la région vertébrale supérieure du dos de l'utilisateur.

4. Support de tête (1) selon la revendication 1, dans lequel ledit élément rigide (2) est une extrusion ou est formé, moulé ou fabriqué avec un matériau en feuille allongé unique pour fournir ladite région s'étendant verticalement (7) et ladite partie incurvée s'étendant vers l'extérieur (8) de celui-ci.

5. Support de tête (1) selon la revendication 4, dans lequel ladite région de support de tête (9) fait saillie de manière latérale à partir de ladite partie incurvée s'étendant vers l'extérieur (8) pour fournir en plus un support sur les côtés de la tête.

6. Support de tête (1) selon la revendication 1, dans lequel ledit harnais (3) est un sac à dos et ladite région s'étendant verticalement (7) possède une région se mettant en prise avec un dispositif de réception de support de tête situé au moins de manière adjacente à une partie supérieure du sac à dos.
